# EUROPÄISCHE PATENTSCHRIFT

(11) **EP 1 446 062 B1**
(45) Veröffentlichungstag und Bekanntmachung des Hinweises auf die Patenterteilung: **10.01.2007**
(21) Anmeldenummer: 02754994.8
(22) Anmeldetag: 13.08.2002
(51) Int. Cl.: A61B 17/72

(54) **DISTRAKTIONSVORRICHTUNG**
DISTRACTION DEVICE
DISPOSITIF DE DISTRACTION

(30) Priorität: 19.11.2001 DE 10156316
(43) Veröffentlichungstag der Anmeldung: 18.08.2004
(73) Patentinhaber: Wittenstein AG, 97999 Igersheim (DE)
(72) Erfinder: WITTENSTEIN, Manfred, 97980 Bad Mergentheim (DE)
(74) Vertreter: Weiss, Peter
(86) Internationale Anmeldenummer: PCT/EP2002/009047
(87) Internationale Veröffentlichungsnummer: WO 2003/043512

(56) Entgegenhaltungen:
- EP-A- 0 919 717
- WO-A-00/01315
- GIROUX E-A ET AL: "ELECTROMAGNETIC HEATING OF A SHAPE MEMORY ALLOY TRANSLATOR" JOURNAL OF PHYSICS D. APPLIED PHYSICS, IOP PUBLISHING, BRISTOL, GB, Bd. 29, Nr. 3, 14. März 1996 (1996-03-14), Seiten 923-928, XP000590791 ISSN: 0022-3727

## Beschreibung

Die vorliegende Erfindung betrifft eine Distraktionsvorrichtung zum Auseinanderbewegen eines ein- oder zweiteiligen ggf. getrennten Knochens zur verlängerung oder Überbrückung eines Knochenspaltes, als ggf. einen in einen Markraum eines Knochen einführbaren Marknagels, welche wenigstens zwei axial gegeneinander bewegbare Elemente aufweist, wobei das erste Element mit dem zweiten Element über zumindest eine Rasteinrichtung zur Sicherung einer Distraktionsbewegung in Verbindung steht.

Derartige Distraktionsvorrichtungen sind in vielfältiger Form und Ausführung im Markt bekannt und gebräuchlich. Sie dienen in erster Linie dazu bei einem Durchtrennen eines Knochen durch eine entsprechende Distraktion eine körpereigene Nachbildung des Knochenmaterials zu verbessern. Eine entsprechende Distraktionsvorrichtung ist bspw. in der EP 0 346 247 B1 beschrieben in welcher ein intrakorporaler Knochenmarknagel aufgezeigt ist, welcher zwei Teile eines Knochens auf mechanische Weise bewegt. Eine derartige Distraktionsvorrichtung ist nicht für sehr kleine Knochen, mit geringen Querschnitten geeignet. Ferner ist aus der DE 39 21 972 C2 ein Marknagel aufgezeigt, welcher ebenfalls auf mechanische Weise verlängerbar ist. Nachteilig ist, bei dem im Stand der Technik bekannten Distraktionsvorrichtungen, dass diese viel zu gross ausgebildet sind und einen schlechten Wirkungsgrad besitzen. Sie können nur begrenzt Distraktionskräfte auf Knochen übertragen.

Ferner ist nachteilig, dass Distraktionsvorrichtungen mit geringen Querschnitt und hohen Distraktionskräften bisher nicht realisierbar sind.

Die EP 0 919 717 A1 (Basis für den Oberbegriff des Anspruchs 1) beschreibt eine Antriebsvorrichtung mit einem aus einer Formgedächtnislegierung geformten Element sowie deren Verwendung. Dort werden zwei Schubmodule gegeneinander bewegt, wobei rein durch entstehende Druckkräfte der Formgedächtnislegierung eine Distraktion erfolgt.

Ein ähnliches Prinzip wird in dem Journal of physics D. applied physics IOP publishing, Bristol, GB Bd. Nr. 29 Nr. 3, vom 14.03.1996, Seiten 923 bis 928, in dem Artikel "Electromagnetic healing of a shape memory alloy translator", beschrieben. Dort erfolgt eine Distraktion zwischen zweier Schubmodule zur Distraktion eines Konsens ebenfalls durch wirkende-Druckkräfte, erzeugt durch eine Formgedächtnislegierung.

Die WO 00/01315 A offenbart eine Distraktionsvorrichtung, bei welcher in einer Arbeitseinrichtung Zugkräfte generiert werden, die über ein Schubelement 5 eine Distraktion auf einen Kolben direkt übertragen.

Der vorliegenden Erfindung liegt daher die Aufgabe zugrunde eine Distraktionsvorrichtung der eingangs genannten Art zu schaffen, welche die genannten Nachteile beseitigt und mit welcher bei äusserst geringen Aussendurchmessern hohe Distraktionskräfte erzielt werden können.

Zur Lösung dieser Aufgabe führt, dass dem zweiten Element ein Schubmodul zur Distraktion des ersten Elementes zugeordnet ist, welches rastbar mit dem zweiten Element in verbindung steht, wobei zwischen Schubmodul und dem zweiten Element ein Federelement vorgesehen ist, welches das Schubmodul druckbeaufschlagt, wobei über eine weitere Rasteinrichtung die Federkraft des Federelementes vom Schubmodul zur Distraktion auf das erste Element übertragbar ist und zum Zurückbewegen des Schubmodules und zum Spannen des Federelementes zwischen Schubmodul und zweitem Element eine Druckstange mit dazwischen eingesetzter Arbeitscinrichtung vorgesehen ist.

Bei der vorliegenden Erfindung ist von wesentlicher Bedeutung, dass einem der beiden Elemente ein Schubmodul zugeordnet ist, welches rastbar mit dem anderen Element in Verbindung steht.

Das Schubmodul weist eine. Arbeitseinrichtung auf, welche mit einer Druckstange in Verbindung steht, um bei entsprechender Betätigung das Schubmodul zum Vorspannen einer Druckfeder, insbesondere eines Federelementes, welches zur Distraktion dient, vorzuspannen.

Hierbei hat sich als besonders vorteilhaft erwiesen, als Arbeitselement Formgedächtniselemente, aus Formgedächtnislegierungen einzusetzen, welche insbesondere bei Erhitzen sich verkürzen, so dass sehr hohe Zugkräfte entstehen. Diese sehr hohen Zugkräfte werden dazu benutzt, das Schubmodul zum Vorspannen des Federelementes zurückzubewegen, wobei die Zurückbewegung rastbar gegenüber dem ersten Element erfolgt, so dass eine anschliessende Distraktion durch das vorgespannte Federelement, welches sich an einem zweiten Element an dessen Wand einerseits und andererseits an einer Stirnseite des Schubmodules abstützt, erfolgt, wenn die Formgedächtniselemente abkühlen und sich wieder entspannen.

Dabei soll ebenfalls im Rahmen der vorliegenden Erfindung liegen, zumindest ein Formgedächtniselement mit entsprechender Heizeinrichtung vorzusehen, wobei die Heizeinrichtung über hier nicht dargestellte Drähte, bspw. induktiv und subkutan mit Energie versorgt wird, um die Arbeitseinrichtung bzw. die Federelemente zu betätigen.

Hierdurch können bei sehr kleinen querschnittlichen Durchmessern Federelemente, insbesondere Druckfedern mit sehr hohen Federkonstanten und Federkräften eingesetzt werden, wobei sehr kleine Formgedächtniselemente mit Heizelementen im Schubmodul vorgesehen sein können, um das Federelement durch Zurückbewegen des Schubmodules mittels der Arbeitseinrichtung vorzuspannen und anschliessend bei Druckbeaufschlagung des Federelementes das Schubmodul mit zusammenwirkender zweiter Rasteinrichtung und das erste Element gegenüber dem zweiten Element auseinander zu bewegen.

Wichtig ist, dass die Formgedächtniselemente durch Zugbeaufschlagung das Schubmodul zum Vorspannen des Federelementes zurückbewegen, so dass hier sehr grosse Vorspannkräfte mittels der Arbeitseinrichtung realisiert werden können Ferner ist auch mittels einer derartigen Bauweise eine Distraktionsvorrichtung von geringer Länge und äusserst geringem Gesamtquerschnitt realisierbar, was ebenfalls im Rahmen der vorliegenden Erfindung liegen soll.

Weitere Vorteile, Merkmale und Einzelheiten der Erfindung ergeben sich aus der nachfolgenden Beschreibung eines bevorzugten Ausführungsbeispieles, dieses zeigt in ihren einzigen Figur eine schematisch dargestellte querschnittliche Darstellung einer erfindungsgemässen Distraktionsvorrichtung R in einer Gebrauchslage.

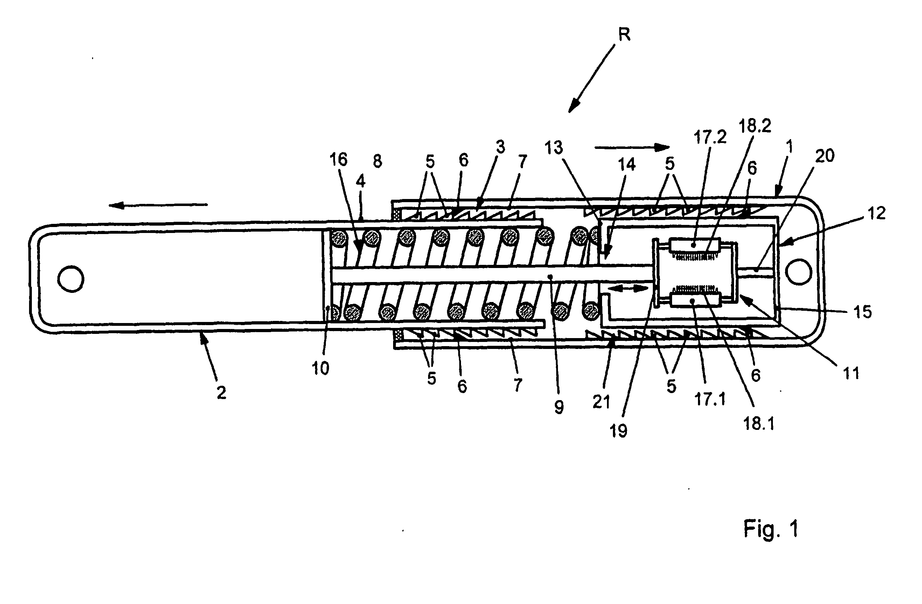

Gemäss Figur 1 weist eine erfindungsgemässe Distraktionsvorrichtung R zwei gegeneinander bewegbare Elemente 1, 2 auf, wobei vorzugsweise das Element 2 rohrförmig in das Element 1 einschiebbar ist. Eine erste Rasteinrichtung 3 weist an einer äusseren Oberfläche 4 des Elementes 2 Rastnasen 5 auf, in welche zumindest eine Sperrklinke 6 des Elementes 1 eingreift. Die Sperrklinke 6 ist vorzugsweise einer Innenwand 7 des Elementes 1 zugeordnet oder aus dieser gebildet.

Die Rasteinrichtung 3 lässt eine Distraktionsbewegung des Elementes 1 und des Elementes 2 auseinander in Richtung der dargestellten Pfeilen zu, jedoch nicht eine Ineinanderbewegung. Dann greift die Sperrklinke 6 zwischen die Rastnasen 5 ein und sperrt eine Ineinanderbewegung von Element 1 zu Element 2. Nur eine Distraktion, d. h. eine Auseinanderbewegung von Element 1 und Element 2 ist durch die Anordnung der Rasteinrichtung 3 gewährleistet.

Eine Dichtung 8 dichtet den gebildeten Ringspalt zwischen Element 1 und Element 2 ab.

Ferner ist wichtig bei der vorliegenden Erfindung, dass innerhalb des Elementes 2 eine Druckstange 9 vorgesehen ist, welche sich an einer Wand 10 im Element 2 abstützt bzw. dort festgelegt ist und vorzugsweise stirnseitig aus dem Element 2 herausgreift.

Endseits der Druckstange 9 schliesst eine Arbeitseinrichtung 11 an, welche endseits mit einem Schubmodul 12 in Verbindung steht.

Das Schubmodul 12 ist vorzugsweise als rohrartiges Element ausgebildet und einends im Bereich einer Stirnseite 13 mit einer Öffnung 14 versehen, durch welche die Druckstange 9 greift.

Innerhalb des Schubmodules 12 ist endseits zwischen der Druckstange 9 und einer Stirnwand 15 die Arbeitseinrichtung 11 eingesetzt.

Ferner ist wichtig bei der vorliegenden Erfindung, dass sich zwischen der Stirnseite 13 des Schubmodules 12 und der Wand 10 des Elementes 2 zumindest ein Federelement 16 erstreckt, welches stirnseitig jeweils an der Wand 10 bzw. der Stirnseite 13 abgestützt ist. Bevorzugt ist das Federelement 16 über die Druckstange 9 axial geführt.

Die Arbeitseinrichtung 11 besteht aus zumindest einem Formgedächtniselement 17.1, 17.2 aus einer Formgedächtnislegierung und zumindest einem diesen zugeordneten Heizelement 18.1, 18.2. Das Heizelement 18.1, 18.2 ist über hier nicht dargestellte elektrische Anschlüsse betätigbar und kann die Formgedächtniselemente 17.1, 17.2 erwärmen.

Dabei können die Heizelemente 18.1, 18.2, bspw. induktiv und subkutan angesteuert werden, um die Arbeitseinrichtung 11 bzw. die aus Formgedächtnislegierungen gebildeten Formgedächtniselemente 17.1, 17.2 zu betätigen.

Die Funktionsweise der vorliegenden Erfindung ist folgende:

Wird mittels des Heizelementes 18.1, 18.2 das Formgedächtniselement 17.1, 17.2 erwärmt, so verkürzen sich die Formgedächtniselemente 17.1, 17.2 erheblich.

Einends sind die Formgedächtniselemente 17.1, 17.2 über eine Aufnahmeplatte 19 abgestützt und ziehen mit den Verbindungselement 20, welches mit der Stirnwand 15 in Verbindung steht, das Schubmodul 12 gegen die Wand 10 bzw. gegen das Federelement 16. Während das Schubmodul 12 gegen die Wand 10 des Elementes 2 beim Erhitzen der Formgedächtniselemente 17.1, 17.2 bewegL wird, verbleibt das Element 1 über die erste Rasteinrichtung 3 fest.

Eine zweite Rasteinrichtung 21 bewirkt, dass in entsprechende an der Innenwand 7 des Elementes 1 vorgesehene Rastnasen 5 zumindest eine Sperrklinke 6 des Schubmodules 12 eingreift. Das Schubmodul 12 lässt sich lediglich mittels der Arbeitseinrichtung 11 bzw. der Formgedächtniselemente 17.1, 17.2 gegen die Wand 10 bzw. gegen das Federelement 16 bewegen und spannt dieses. Ein Zurückbewegen wird über die zweite Rasteinrichtung 21 bzw. über die Sperrklinke 6 und die darin eingreifenden Rastnasen 5, verhindert. Auf diese Weise lässt sich sehr einfach und effektiv das Federelement 16 vorspannen, welches bei Abkühlen und entsprechendem Verlängern der Arbeitseinrichtung 11 bzw. der Formgedächtniselement 17.1, 17.2 eine Distraktion des Elementes 1 gegenüber dem Element 2 zu lässt, in dem das Federelement 16 gegen die Stirnseite 13 des Schubmodules 12 vorgespannt und druckbeaufschlagt ist, und das Schubmodul 12 über die Sperrklinke 6 das erste Element 1 gegenüber dem zweiten Element 2 zur Distraktion auseinander bewegt.

Nach einer entsprechenden Distraktion kann dann wieder, wie oben beschrieben, durch Verkürzen der Arbeitseinrichtung 11 bzw. der Formgedächtniselemente 17.1, 17.2 das Schubmodul 12 gegenüber dem Element 1 in oben beschriebener Weise zurückbewegt werden, um das Federelement 16 wieder vorzuspannen. Dieser Vorgang kann sich beliebig oft wiederholen.

Von Vorteil ist bei der vorliegenden Erfindung, dass sehr geringe Durchmesser von Distraktionsvorrichtungen R realisiert werden können und sehr starke Federelemente 16 mit einer hohen Federkonstante eingesetzt werden können.

Ferner können durch Verkürzung der Formgedächtniselemente 17.1, 17.2 sehr hohe Zugkräfte zum Zurückbewegen des Schubmodules 12 bzw. zum Vorspannen des Federelementes 16 erzeugt werden, was ebenfalls zur Minimierung des Gesamtdurchmessers der Distraktionsvorrichtung R erheblich beiträgt.

### Positionszahlenliste

| | | | | | |
|---|---|---|---|---|---|
| 1 | Element | 34 | | 67 | |
| 2 | Element | 35 | | 68 | |
| 3 | Rasteinrichtung | 36 | | 69 | |
| 4 | Oberfläche | 37 | | 70 | |
| 5 | Rastnase | 38 | | 71 | |
| 6 | Sperrklinke | 39 | | 72 | |
| 7 | Innenwand | 40 | | 73 | |
| 8 | Dichtung | 41 | | 74 | |
| 9 | Druckstange | 42 | | 75 | |
| 10 | Wand | 43 | | 76 | |
| 11 | Arbeitseinrichtung | 44 | | 77 | |
| 12 | Schubmodul | 45 | | 78 | |
| 13 | Stirnseite | 46 | | 79 | |
| 14 | Öffnung | 47 | | | |
| 15 | Stirnwand | 48 | | | |
| 16 | Federelement | 49 | | R | Distraktionsvorrichtung |
| 17 | Formgedächtniselement | 50 | | | |
| 18 | Heizelement | 51 | | | |
| 19 | Aufnahmeplatte | 52 | | | |
| 20 | Verbindungselement | 53 | | | |
| 21 | Rasteinrichtung | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Distraktionsvorrichtung zum Auseinanderbewegen eines ein- oder zweiteiligen ggf. getrennten Knochens zur Verlängerung oder Überbrückung eines Knochenspaltes, als ggf. einen in einen Markraum eines Knochen einführbaren Marknagels, welche wenigstens zwei axial gegeneinander bewegbare Elemente (1, 2) aufweist, wobei das erste Element (1) mit dem zweiten Element (2) über zumindest eine Rasteinrichtung (3) zur Sicherung einer Distraktionsbewegung in Verbindung steht, wobei dem zweiten Element (2) ein Schubmodul (12) zur Distraktion des ersten Elementes (1) zugeordnet ist, welches rastbar mit dem zweiten Element (2) in Verbindung steht, wobei zwischen Schubmodul (12) und dem zweiten Element (2) ein Federelement (16) vorgesehen ist, welches das Schubmodul (12) druckbeaufschlagt, wobei über eine weitere Rasteinrichtung (21) die Federkraft des Federelementes (16) vom Schubmodul (12) zur Distraktion auf das erste Element (1) übertragbar ist und zum Zurückbewegen des Schubmodules (12) und zum Spannen des Federelementes (16) zwischen Schubmodul (12) und zweitem Element (2) eine Arbeitseinrichtung (11) vorgesehen ist, wobei die Arbeitseinrichtung (11) aus zumindest einem Formgedächtniselement (17.1, 17.2) und einer Heizeinrichtung (18.1, 18.2) gebildet ist, durch Beheizen des Formgedächtniselementes (17.1, 17.2) mittels der Heizeinrichtung (18.1, 18.2) ein Verkürzen des Formgedächtniselementes (17.1, 17.2) erfolgt, was ein Zurückbewegen des Schubmodules (12) gegenüber dem ersten Element (1) zur Folge hat, wobei eine Bewegung des ersten Elementes (1) über die erste Rasteinrichtung (3) gegenüber dem zweiten Element (2) gesperrt ist, **dadurch gekennzeichnet, dass** zwischen Schubmodul (12) und zweitem Element (2), zusammen mit der Arbeitseinrichtung (11), ein Druckstange (9) vorgesehen ist.

2. Distraktionsvorrichtung nach Anspruch 1; **dadurch gekennzeichnet, dass** die zweite Rasteinrichtung (21) eine Distraktionsbewegung des Schubmodules (12) auf das erste Element (1) sperrend überträgt und zur Distraktion des ersten Elementes (1) gegenüber dem zweiten Element (2) eine Distraktionsbewegung überträgt, wobei das Element (1) an dem Element (2) mittels der ersten Rasteinrichtung (3) rastbar entlangfahrbar ist.

3. Distraktionsvorrichtung nach Anspruch 2, **dadurch gekennzeichnet, dass** die Arbeitseinrichtung (11) innerhalb des Schubmodules (12) angeordnet ist, wobei die Druckstange (9) einends das Schubmodul (12) durchgreift.

4. Distraktionsvorrichtung nach wenigstens einem der Ansprüche 1 bis 3, **dadurch gekennzeichnet, dass** nach einem Abkühlen des Formgedächtniselementes (17.1, 17.2) eine Verlängerung der Arbeitseinrichtung (11) erfolgt und durch Druckbeaufschlagung des Federelementes (16) vom zweiten Element (2) auf das Schubmodul (12) eine Distraktion des ersten Elementes (1) gegenüber dem zweiten Element (2) erfolgt.

5. Distraktionsvorrichtung nach wenigstens einem der Ansprüche 1 bis 4, **dadurch gekennzeichnet, dass** wenigstens zwei Formgedächtniselemente (17.1, 17.2) mit jeweils einer Heizeinrichtung (18.1, 18.2) zwischen Druckstange (9) und des Schubmodules (12) angeordnet sind.

6. Distraktionsvorrichtung nach wenigstens einem der Ansprüche 1 bis 5, **dadurch gekennzeichnet, dass** durch Beheizen zumindest eines Formgedächtniselementes (17.1, 17.2) das Schubmodul (12) gegen das Federelement (16) bewegbar ist, dieses gegen das zweite Element (2) verfährt und damit das Federelement (16) spannt, wobei eine Rückbewegung des Schubmodules (12) in Distraktionsrichtung mittels der zweiten Rasteinrichtung (21) gesperrt ist.

7. Distraktionsvorrichtung nach wenigstens einem der Ansprüche 1 bis 6 **dadurch gekennzeichnet, dass** das Federelement (16) die zumindest eine Druckstange (9) umfängt und stirnseitig an dem Schubmodul (12) einends und andernends am zweiten Element (2) abgestützt ist.

8. Distraktionsvorrichtung nach wenigstens einem der Ansprüche 1 bis 7, **dadurch gekennzeichnet, dass** das zumindest eine Formgedächtniselement (17.1, 17.2) einends an der Druckstange (9) und andernends direkt am Schubmodul (12) oder an einer Stirnseite (13) des Schubmodules (12) abgestützt ist.

## Claims

1. Distraction device for moving apart a one- or two-part, optionally severed bone in order to extend or bridge a bone gap, optionally in the form of a medullary pin, which is insertable into a medullary space of a bone and comprises at least two elements (1, 2) that are movable axially towards one another, wherein the first element (1) is connected to the second element (2) by at least one detent device (3) for securing a distraction movement, wherein associated with the second element (2) for distraction of the first element (1) is a translator module (12), which is connected latchably to the second element (2), wherein provided between translator module (12) and the second element (2) is a spring element (16) that exerts pressure on the translator module (12), wherein via a further detent device (21) the spring force of the spring element (16) is transmissible from the translator module (12) for distraction to the first element (1) and for the return movement of the translator module (12) and for the tensioning of the spring element (16) a working device (11) is provided between translator module (12) and second element (2), wherein the working device (11) is formed from at least one shape memory element (17.1, 17.2) and one heating device (18.1, 18.2), by virtue of heating the shape memory element (17.1, 17.2) by means of the heating device (18.1, 18.2) a shortening of the shape memory element (17.1, 17.2) occurs, thereby leading to a return movement of the translator module (12) relative to the first element (1), wherein a movement of the first element (1) relative to the second element (2) is blocked by means of the first detent device (3), **characterized in that** between translator module (12) and second element (2), together with the working device (11), a push rod (9) is provided.

2. Distraction device according to claim 1, **characterized in that** the second detent device (21) in a blocking manner transmits a distraction movement of the translator module (12) to the first element (1) and for distraction of the first element (1) relative to the second element (2) transmits a distraction movement, wherein the element (1) is latchably followable relative to the element (2) by means of the first detent device (3) .

3. Distraction device according to claim 2, **characterized in that** the working device (11) is disposed inside the translator module (12), wherein the push rod (9) at one end engages through the translator module (12).

4. Distraction device according to at least one of claims 1 to 3, **characterized in that** after cooling of the shape memory element (17.1, 17.2) a lengthening of the working device (11) occurs and by virtue of pressure loading of the spring element (16) of the second element (2) upon the translator module (12) a distraction of the first element (1) relative to the second element (2) occurs.

5. Distraction device according to at least one of claims 1 to 4, **characterized in that** at least two shape memory elements (17.1, 17.2) each with a heating device (18.1, 18.2) are disposed between push rod (9) and the translator module (12).

6. Distraction device according to at least one of claims 1 to 5, **characterized in that** by virtue of heating at least one shape memory element (17.1, 17.2) the translator module (12) is movable towards the spring element (16), the spring element (16) travels towards the second element (2) and hence tensions the spring element (16), wherein a return movement of the translator module (12) in distraction direction is blocked by means of the second detent device (21).

7. Distraction device according to at least one of claims 1 to 6 **characterized in that** the spring element (16) surrounds the at least one push rod (9) and is supported by one end face against the translator module (12) and by the other end face against the second element (2).

8. Distraction device according to at least one of claims 1 to 7, **characterized in that** the at least one shape memory element (17.1, 17.2) is supported by one end against the push rod (9) and by the other end against the translator module (12) or against an end face (13) of the translator module (12).

## Revendications

1. Dispositif de distraction destiné à écarter un os en une ou deux parties, éventuellement séparé, en vue d'allonger ou de ponter une fissure d'os, éventuellement sous forme d'un clou centro-médullaire pouvant être introduit dans un espace centro-médullaire, présentant au moins deux éléments (1, 2) déplaçables axialement l'un par rapport à l'autre, le premier élément (1) étant relié au deuxième élément (2) par l'intermédiaire d'au moins un dispositif d'encliquetage (3) pour empêcher un mouvement de distraction, au deuxième élément (2) étant associé un module de glissement (12) destiné à :a distraction du premier élément (1) qui est relié de manière encliquetable au deuxième élément (2), entre le module de glissement (12) et le deuxième élément (2) étant prévu un élément de ressort (16) qui soumet le module de glissement (12) à de la pression, la force de ressort de l'élément de ressort (16) pouvant être transmise, par l'intermédiaire d'un autre dispositif d'encliquetage (21), du module de glissement (12) au premier élément (1) pour la distraction et que pour le mouvement de recul du module de glissement (12) et la tension de l'élément de ressort (16) entre le module de glissement (12) et le deuxième élément (2) est prévu un dispositif de travail (11), le dispositif de travail (11) étant formé d'au moins un élément à mémoire de forme (17.1, 17.2) et d'un dispositif chauffant (18.1, 18.2), par le chauffage de l'élément à mémoire de forme (17.1, 17.2) au moyen du dispositif chauffant (18.1, 18.2) ayant lieu un raccourcissement de l'élément à mémoire de forme (17.1, 17.2), ce qui a pour conséquence un mouvement de recul du module dé glissement (12) par rapport au premier élément (1), un mouvement du premier élément (1), par l'intermédiaire du premier dispositif d'encliquetage (3), par rapport au deuxième élément (2) étant bloqué,
**caractérisé par le fait qu'**entre le module de glissement (12) et le deuxième élément (2) est prévue, ensemble avec le dispositif de travail (11), une tige de poussée (9).

2. Dispositif de distraction selon la revendication 1, **caractérisé par le fait que** le deuxième dispositif d'encliquetage (21) transmet de manière bloquante un mouvement de distraction du module de glissement (12) au premier élément (1) et transmet un mouvement de distraction pour la distraction du premier élément (1) par rapport au deuxième élément (2), l'élément (1) pouvant être déplacé de manière encliquetable par rapport à l'élément (2) au moyen du premier dispositif (3).

3. Dispositif de distraction selon la revendication 2, **caractérisé par le fait que** le dispositif de travail (11) est disposé dans le module de glissement (12), la tige de poussée (9) traversant, à une extrémité, le module de glissement (12).

4. Dispositif de distraction selon au moins l'une des revendications 1 à 3, **caractérisé par le fait qu'**après un refroidissement de l'élément à mémoire de forme (17.1, 17.2) a lieu un allongement du dispositif de travail (11) et que par soumission de l'élément de ressort (16) à de la pression du deuxième élément (2) au module de glissement (12) a lieu une distraction du premier élément (1) par rapport au deuxième élément (2).

5. Dispositif de distraction selon au moins l'une des revendications 1 à 4, **caractérisé par le fait qu'**au moins deux éléments à mémoire de forme (17.1, 17.2) sont disposés avec, chacun, un dispositif chauffant (18,1, 18.2) entre la tige de poussée (9) et le module de glissement (12).

6. Dispositif de distraction selon au moins l'une des revendications 1 à 5, **caractérisé par le fait que** par chauffage d'au moins un élément à mémoire de forme (17.1, 17.2) peut être déplacé le module de glissement (12) par rapport à l'élément de ressort (16), ce dernier se déplace par rapport au deuxième élément (2) et tend, de ce fait, l'élément de ressort (16), un recul du module de glissement (12) en direction de distraction étant bloqué au moyen du deuxième dispositif d'encliquetage (21).

7. Dispositif de distraction selon au moins l'une des revendications 1 à 6, **caractérisé par le fait que** l'élément de ressort (16) entoure l'au moins une tige de poussée (9) et s'appuie, du côté frontal, sur le module de glissement (12), à une extrémité, et, à l'autre extrémité sur le deuxième élément (2).

8. Dispositif de distraction selon au moins l'une des revendications 1 à 7, **caractérisé par le fait que** l'au moins un élément à mémoire de forme (17.1, 17.2) s'appuie, à une extrémité, sur la tige de poussée (9) et, à l'autre extrémité, directement sur le module de glissement (12) ou sur une face frontale (13) du module de glissement (12).
